# EUROPEAN PATENT SPECIFICATION

(11) **EP 3 821 902 B1**
(45) Date of publication and mention of the grant of the patent: **22.02.2023**
(21) Application number: 19834655.3
(22) Date of filing: 16.05.2019
(51) Int. Cl.: A61K 36/896, A61K 36/54, A61K 36/704, A61K 36/185, A61P 31/20, A61P 15/02, A61P 15/00, A61K 9/02, A61K 9/06, A61K 9/00, A61K 36/88, A61K 36/90, A61P 31/04, A61P 31/12

(54) **TRADITIONAL CHINESE MEDICINE COMPOSITION, TRADITIONAL CHINESE MEDICINE COMPOUND PREPARATION AND PREPARATION METHOD AND USE THEREOF**
TRADITIONELLE CHINESISCHE ARZNEIMITTELZUSAMMENSETZUNG, TRADITIONELLE CHINESISCHE ARZNEIMITTELZUBEREITUNG SOWIE VERFAHREN ZU IHRER HERSTELLUNG UND IHRE VERWENDUNG
COMPOSITION DE MÉDECINE CHINOISE TRADITIONNELLE, PRÉPARATION DE COMPOSÉ DE MÉDECINE CHINOISE TRADITIONNELLE ET SON PROCÉDÉ DE PRÉPARATION ET SON UTILISATION

(30) Priority: 12.07.2018 CN 201810765903
(43) Date of publication of application: 19.05.2021
(73) Proprietor: Jiangsu Zhengyang Pharmaceutical Co., Ltd., Nanjing, Jiangsu 211300 (CN)
(72) Inventor: RONG, Ru Cheng, Nanjing, Jiangsu 211300 (CN)
(74) Representative: Niburska, Danuta
(86) International application number: PCT/CN2019/087286
(87) International publication number: WO 2020/010919

(56) References cited:
- CN-A- 102 743 662
- CN-A- 102 908 559
- DATABASE WPI Week 201322 Thomson Scientific, London, GB; AN 2013-B12193 XP002803608, & CN 102 743 662 A (UNIV GUANGXI TRADITIONAL CHINESE MEDICAL) 24 October 2012 (2012-10-24)
- DATABASE WPI Week 201840 Thomson Scientific, London, GB; AN 2018-42388W XP002803609, & CN 108 066 672 A (GUANGXI WONDER PHARM CO LTD) 25 May 2018 (2018-05-25)
- DATABASE WPI Week 201831 Thomson Scientific, London, GB; AN 2018-32918N XP002803610, & CN 107 929 619 A (GUANGXI WONDER PHARM CO LTD) 20 April 2018 (2018-04-20)
- DATABASE WPI Week 201432 Thomson Scientific, London, GB; AN 2014-E98191 XP002803611, & CN 103 520 592 A (ZHANG L) 22 January 2014 (2014-01-22)
- DATABASE WPI Week 201468 Thomson Scientific, London, GB; AN 2014-S51829 XP002803612, & CN 103 933 429 A (SUN Y) 23 July 2014 (2014-07-23)
- Ma Xiaoping: "Research Progress of Traditional Chinese Medicine in Prevention and Treatment of Cervical HPV Infection", Journal of Sichuan of Traditional Chinese Medicine, vol. 35, no. 6, 31 December 2017 (2017-12-31), pages 215-217, XP009525872, ISSN: 1000-3649
- MIAO Wen-jin: "Clinical Observation on Treating Condyloma Acuminatum by Dayflower Washing Combined with COz Laser Therapy", Clinical Journal of Chinese Medicine, vol. 5, no. 23, 31 December 2013 (2013-12-31), pages 20-21, XP055777759,

## Description

The disclosure relates to the field of traditional Chinese medicine, and more particularly, to a traditional Chinese medicine composition for treatment of Human papillomavirus (HPV) infection and a preparation method thereof.

HPV is a member of the *Papillomaviridae* family of viruses that specifically infect human epithelial cells. Over 100 HPV types have been described. About 30-40 HPV types may spread through broken skin or mucous membranes. Infection with HPV is so common that nearly 70-80% of people who are sexually-active will get HPV at some time in their life.

In recent years, the rate of female genital HPV infection has continued to increase. Studies have shown a close relationship between low-risk HPV types and genital warts on a female. About a dozen HPV types are called high-risk types because persistent infection has been linked to precancerous lesions and cervical cancer. These high-risk HPV types cause about 99.7% of cervical cancer. World Health Organization (WHO) has said almost all cervical cancer cases (100%) are linked to infection with high-risk HPV. Most people who have had high-risk HPV infections are more likely to have persistent HPV infection, a more rapid progress to precancerous lesion and invasion cervical cancer. Cervical cancer typically develops from precancerous changes over 10-15 years. HPV vaccines that prevent infection with certain HPV subtypes are most effective when given before exposure to any HPV. These vaccines cannot help treat or protect against types of HPV to which the person has already been exposed. There is an urgent need to develop new drugs or approaches that effectively treat HPV infection in female reproductive tract, clear up persistent HPV infections, prevent and treat precancerous lesion to reduce risk of cervical cancer.

Currently, there is no effective HPV therapeutic medications for safe use in the females suffering from high-risk HPV infections of the reproductive tract. No standardized and unified treatment plan are clinically approved to treat persistent high-risk HPV infection in the cells of cervix. The main clinical treatment for HPV infection is physical therapy and chemotherapy. The physical therapy involves the use of a laser beam, a wire loop heated by electrical current (LEEP procedure), electrocautery, etc. The chemotherapy uses interferon to inhibit viral replication. When these treatments affect normal cells and produce side effects.

In recent years, researchers have realized that traditional Chinese medicine has considerable potential for research and development of new drugs against HPV infection. But failures arise from a lack of multi-center clinical trials with large sample sizes. Although some Chinese herbal medicine ingredients exfoliate cervical cells from cervix, they generally cause pain, display poor therapeutic effects and high toxicity.

CN 1027-43662 A discloses a pharmaceutical composition for treating gynaecological inflammation. The composition consists of: between 15 and 60 weight parts of Smilax Glabra Root, between 9 and 15 weight parts of Rhizoma Polygoni Cuspidati, between 4.5 and 9 weight parts of Radix Sophorae Flavescentis, between 3 and 10 weight parts of Cnidii Fructus, between 15 and 30 weight parts of Chinese Knotweed, between 10 and 15 weight parts of Rhizoma Smilacis Chinensis, between 9 and 15 weight parts of Caulis Sargentodoxae, between 9 and 15 weight parts of Fructus Kochiae, between 9 and 15 weight parts of Herba Violae, and between 3 and 12 weight parts of Phellodendri Chinensis Cortex. CN 108066672 A discloses an emulsion for treating gynaecological inflammation. The emulsion is made from the following: between 20 and 50 weight parts of Chinese Knotweed, between 10 and 30 weight parts of Smilax Glabra Root, between 10 and 20 weight parts of Acorus gramineus, between 10 and 30 weight parts of Ovateleaf Holly Bark, between 10 and 30 weight parts of Moutan Cortex Radicis, between 10 and 20 weight parts of wild chrysanthemum, between 10 and 20 weight parts of Phellodendri Chinensis Cortex, between 1 and 3 weight parts of borneol, between 15 and 25 weight parts of Tween-80, and between 1 and 3 weight parts of chlorhexidine acetate. CN 107929619 A discloses a lotion for treating gynaecological inflammation. The lotion is made from the following: between 20 and 50 weight parts of Chinese Knotweed, between 10 and 30 weight parts of Smilax Glabra Root, between 10 and 20 weight parts of Acorus gramineus, between 10 and 30 weight parts of Ovateleaf Holly Bark, between 10 and 30 weight parts of Moutan Cortex Radicis, between 10 and 20 weight parts of wild chrysanthemum, and between 10 and 20 weight parts of Phellodendri Chinensis Cortex. CN 103520592 A discloses a decoction for the treatment of chronic pelvic pain. The decoction is made from the raw materials consisting of: between 11 and 14 wt.% of Prunellae Spica, between 9 and 11 wt.% of Smilax Glabra Root, between 7 and 9 wt.% of Euryales Semen, between 6 and 8 wt.% of lithospermum root, between 5 and 7 wt.% of Toosendan Fructus, between 6 and 8 wt.% of Ramulus Cinnamomi, between 3 and 5 wt.% of Moutan Cortex Radicis, between 4 and 6 wt.% of Herba Leonuri, between 5 and 7 wt.% of Bupleuri Radix, between 5 and 7 wt.% of Herba Speranskiae Tuberculatae, between Persicae Semen, between 3 and 5 wt.% of Liquidambaris Fructus, between 4 and 6 wt.% of Carthami Flos, between 7 and 9 wt.% of Pyrrosiae Folium, and between 6 and 8 wt.% of Notopterygii Rhizoma et Radix. CN 103933429 A recites a composition for the treatment of colpitis. The composition consists of: between 10 and 50 g of Herba Bidentis Bipinnatae, between 10 and 45 g of Medulla Stachyuri, between 30 and 60 g of Polygonum tinctorium Ait, between 20 and 50 g of Smilax Glabra Root, between 15 and 35 g of Herba Salviae Chinensis, between 10 and 30 g of Herba Spirodelae, between 10 and 35 g of Rubus cochinchinensis, between 15 and 35 g of Commelina Communis, between 20 and 50 g of Herba Verbenae, between 15 and 60 g of Herba Leonuri, between 10 and 30 g of leaves of Prunus persica, and between 10 and 30 g of Radix Glycyrrhizae.

It is an object of the disclosure to provide a traditional Chinese medicine composition for treatment of HPV infection and associated diseases including vaginitis, cervicitis, and cervical ectropion caused by HPV.

The traditional Chinese medicine composition comprises an extract of: 30-60 parts by weight of Smilax Glabra Root, 10-20 parts by weight of Cinnamon Twig, 10-20 parts by weight of Chinese Knotweed, and 10-20 parts by weight of Commelina Communis.

In a class of this embodiment, the composition comprises an extract of: 30-55 parts by weight of Smilax Glabra Root, 12-18 parts by weight of Cinnamon Twig, 10-18 parts by weight of Chinese Knotweed and 12-18 parts by weight of Commelina Communis.

The disclosure also provides a pharmaceutical preparation comprising the traditional Chinese medicine composition.

The pharmaceutical preparation has a dosage form of creams, lotions, gels, or suppositories.

The pharmaceutical preparation is prepared by pulverizing the traditional Chinese medicine composition into decoction pieces, dissolving the decoction pieces in ethanol, obtaining an extract, concentrating and drying the extract at low temperature.

The pharmaceutical preparation is prepared by dissolving the traditional Chinese medicine composition in water, extracting, and refining by a macroporous resin.

The pharmaceutical preparation is prepared by dissolving the traditional Chinese medicine composition in ethanol, extracting, and refining by a macroporous resin.

A method for preparing the pharmaceutical preparation comprises: evenly mixing raw materials, dissolving the raw materials in a solvent, obtaining an extract, and concentrating the extract to obtain a pharmaceutical cream.

The solvent used for extraction is ethanol or water, and the effective ingredients of the extract are concentrated under reduced pressure.

Specifically, a method of preparing the pharmaceutical preparation comprises:
1) mixing the raw materials to yield a mixture;
2) dissolving the mixture in an ethanol solution, decocting the mixture dissolved in the ethanol solution, thus yielding a first filtrate and a first residue;
3) collecting the first filtrate, dissolving the first residue in the ethanol solution, and decocting the first residue dissolved in the ethanol solution, thus yielding a second filtrate and a second residue;
4) repeating 3), mixing collected filtrates, and condensing the filtrates to yield the cream having a relative density of 1.10-1.40 at 60°C.

The extraction process includes the decoction and filtering of the raw material or filter residue.

In a class of this embedment, the ethanol solution is replaced by water.

In a class of this embedment, the ethanol solution has a concentration of 50-80% (v/v), and a volume weight ratio of the ethanol solution to the mixture is 5-15.

In a class of this embedment, in 2), the mixture and the filtrates are decocted in the ethanol solution at 50-75°C for 2-5 hours.

In a class of this embedment, in 4), repeating 3) for 2-5 times, and the filtrates are condensed at 50-80°C.

Specifically, the preparation method comprises:
1) mixing and pulverizing the Smilax Glabra Root, Cinnamon Twig, Chinese Knotweed, and Commelina Communis into decoction pieces;
2) dissolving the decoction pieces in 60% ethanol, the volume of which is 10 folds of the mass of the decoction pieces, filtering the decoction pieces, collecting a first filtrate, dissolving a filter residue in 60% ethanol, the volume of which is 10 folds of the mass of the filter residue, filtering the filter residue, collecting a second filtrate, repeating operations of dissolving and filtering for 2-3 times with each dissolving for 1-2 hours, and mixing collected filtrates; and
3) concentrating the collected filtrates at 60°C under reduced pressure to yield a pharmaceutical cream with a relative density of 1.10-1.40.

Specifically, the preparation method comprises the following steps of:
1) mixing and pulverizing the Smilax Glabra Root, Cinnamon Twig, Chinese Knotweed, and Commelina Communis into decoction pieces;
2) dissolving the decoction pieces in water, the volume of which is 12 folds of the mass of the decoction pieces, filtering the decoction pieces, collecting a first filtrate, dissolving a filter residue in water, the volume of which is 12 folds of the mass of the filter residue, filtering the filter residue, collecting a second filtrate, repeating operations of dissolving and filtering with each dissolving for 1-2 hours, and mixing collected filtrates;
3) adsorbing the collected filtrates in 2) on an AB-8 macroporous absorbent resin, eluting the resin consecutively with water and 75% ethanol, and colleting an eluent comprising 75% ethanol;
4) concentrating the eluent comprising 75% ethanol in 3) at 60°C under reduced pressure to yield a pharmaceutical cream with a relative density of 1.10-1.40.

Specifically, the preparation method comprises the following steps of:
1) mixing and pulverizing the Smilax Glabra Root, Cinnamon Twig, Chinese Knotweed, and Commelina Communis into decoction pieces;
2) dissolving the decoction pieces with 60% ethanol, the volume of which is 10 folds of the mass of the decoction pieces, filtering the decoction pieces, collecting a first filtrate, repeating operations of dissolving and filtering for 2 times with each dissolving for 1-2 hours, and mixing collected filtrates;
3) adsorbing the collected filtrates in 2) on an AB-8 macroporous absorbent resin, eluting the resin consecutively with water and 75% ethanol, and colleting an eluent comprising 75% ethanol; and
4) concentrating the eluent comprising 75% ethanol in 3) at 60°C under reduced pressure to yield a pharmaceutical cream with a relative density of 1.10-1.40.

In a class of this embodiment, the filtrates are obtained by reflux extraction and then mixed.

In a class of this embodiment, the pharmaceutical cream is mixed with an excipient to form a gel or a suppository.

In a class of this embodiment, the pharmaceutical cream is mixed with an excipient to form a lotion.

The disclosure provides a method for prevention and/or treatment of human papillomavirus (HPV) infection comprising administering a patient in need thereof the pharmaceutical preparation.

The disclosure provides a method for prevention and/or treatment of vaginitis, cervicitis, or cervical ectropion comprising administering a patient in need thereof the pharmaceutical preparation. Specifically, the pharmaceutical preparation is used for treatment of HPV infection and associated diseases including vaginitis, cervicitis, and cervical ectropion caused by HPV.

The advantages associated with the pharmaceutical preparation of the disclosure include but are not limited to fewer and mild side effects, convenient use, and safety over conventional drugs used to treat HPV infection and associated diseases, such as cervical intraepithelial neoplasia (CIN) 1 and CIN 2. The clinical trials show that the probability of success of the pharmaceutical preparation for treatment of HPV infection and associated diseases exceeds 85%.

The traditional Chinese medicine composition of the disclosure contains effective active ingredients with less toxic side effects, such as flavonoids and alkaloids. Studies have confirmed that the traditional Chinese medicinal composition is able to kill HPV and human cervical cancer HeLa cells, and to treat cervicitis, which is suitable for treatment of HPV infection and the associated diseases, such as vaginitis, cervicitis, and cervical ectropion. The dosage forms of the pharmaceutical preparation, including gel, suppository and lotion, are easy for patients to use and improve patient compliance with medication. The pharmaceutical preparation with the dosage forms can stay in the vagina for a long time and penetrate into the mucosal folds, which helps the drug to contact the lesion and pathogenic factors, and facilitates the drug absorption.

Each component of the traditional Chinese medicine composition of the disclosure provides the following pharmacological effects:

In Traditional Chinese Medicine, Smilax Glabra Root is dried rhizome and root of Smilax Glabra that belongs to family *Smilacaceae.* Smilax Glabra Root tastes Sweet and belongs to the 'Herbs that dispels Dampness and relieve Toxicity' category. As suggested by the category Smilax Glabra Root is Neutral in nature and thought to target the Stomach and the Liver. "Neutral" means that Smilax Glabra Roots typically do not affect the balance in your body. The primary conditions or symptoms for which Smilax Glabra Root may be prescribed by TCM doctors to treat dysuria, leucorrhoea, carbuncle, scrofula, and scabies. Smilax glabra root mainly contains flavonoids, tannins, and resins, such as astilbin, isoengelitin, 3,5,4'-trihydroxystilbene, L-epicatechin, and succinic acid.

In Traditional Chinese Medicine, Cinnamon Twigs are dried young branches of Chinese Cinnamon that belongs to the family *Lauraceae.* Cinnamon Twigs taste Pungent and Sweet, and belong to the 'Warm/Acrid herbs that release the Exterior' category. As suggested by the category Cinnamon Twigs are plants that are Warm in nature and thought to target the Lung, the Heart, and the Bladder. "Warm" means that Cinnamon Twigs tend to help people who have too much "cold" in their body. The main actions of Cinnamon Twigs according to TCM: relieves the Exterior through sweating, disperse Cold and relieve Pain, removes obstruction of Yang and promotes the circulation of Yang Qi in the chest. The primary conditions or symptoms for which Cinnamon Twigs may be prescribed by TCM doctors: common cold, abdominal pain, dysmenorrhea, palpitations, edema, and phlegm fluid accumulation. Cinnamon Twigs contains volatile oils such as cinnamaldehyde and cinnamic acid, as well as phenols, organic acids, glycosides, coumarin and other ingredients.

In Traditional Chinese Medicine, Chinese Knotweed is the aerial part of Chinese Knotweed that belongs to the family *Polygonaceae.* The whole plant can be applied for medical use. Chinese Knotweed is Cold in nature and tastes bitter and sour. "Cold" means that Chinese Knotweed typically help people who have too much "heat" in their body. The main actions of Chinese Knotweed according to TCM includes: clear Heat and release Toxicity, drain Dampness, cool blood and stop itching, remove nebula and improve the visual acuity. The primary conditions or symptoms for which Chinese Knotweed may be prescribed by TCM doctors: dysentery, dyspepsia, hepatitis, diphtheria, whooping cough, corneal clouding, fungal vaginitis, leucorrhea, furunculosis, pustules in children, eczema, snake bites. Chinese Knotweed mainly contains indigo glycosides, polygonatum, p-coumaric acid, ferulic acid, vanillic acid, protocatechuic acid, caffeic acid, etc.

In Traditional Chinese Medicine, Commelina Communis is a perennial herbaceous plant that belongs to the family *Commelinaceae.* The aerial part can be applied for medical use. Commelina Communis is Cold in nature and tastes slightly bitter and sweet. The main actions of Chinese Knotweed according to TCM includes: clears Heat, releases Toxicity, and induces urination. The primary conditions or symptoms for Commelina Communis may be prescribed by TCM doctors: edema, tinea pedis, dysuria, cold, erysipelas, mumps, jaundice hepatitis, fever dysentery, malaria, epistaxis, hematuria, bleeding, leucorrhea, sore throat, carbuncle and furuncle. Commelina Communis mainly contains protocatechuic acid, caffeic acid, p-hydroxybenzoic acid, methyl gallate, coylidin-7-O-β-D-glucoside, isoquercetin, isovitexin, etc.

In the prescription of the disclosure, Smilax Glabra Root is a Jun (sovereign) ingredient drug used to target the disease or main symptoms suffered. Cinnamon Twigs and Chinese Knotweed are Chen (minister) ingredient drugs used to assistant Jun to dispels dampness, and relieve toxicity for detumescence. Commelina Communis is a Zuo (assistant) ingredient drug that help to relive and eliminate toxicities or side effects caused by Jun and Chen drugs. The traditional Chinese medicine composition of the disclosure protects females against diseases caused by HPV, such as genital warts, genital ulcer, problems urinating, as well as vaginal itching and pain.

Smilax glabra root contains two main active ingredients: flavonoids and tannins. Cinnamon Twig contains phenylpropanoids such as cortex aldehydes and cinnamic acid. Chinese Knotweed contains organic acids such as indoxyl sulfate. Commelina Communis contains organic acids and flavonoids. The above effective ingredients can be extracted from the raw materials using ethanol, while removing the macromolecules insoluble in ethanol and involved in allergic reactions, such as protein and glycoprotein. Therefore, the disclosure improves safety on active pharmaceutical ingredients extracted from the raw materials.

Optionally, the above effective ingredients can be extracted by dissolving the raw materials in water, and eluting the extract with the macroporous absorbent resin. The extraction method can also remove the above macromolecules involved in allergic reactions, enhance drug safety, provides a low-cost water extraction process with minimal impurity levels, thereby achieving a significantly greater therapeutic effect in a patient.

To further illustrate the disclosure, embodiments detailing a traditional Chinese medicine composition are described below. It should be noted that the following embodiments are intended to describe and not to limit the disclosure.

### Example 1

A traditional Chinese medicine composition for treatment of HPV infection comprises following raw materials: 40 parts by weight of smilax glabra root, 15 parts by weight of Cinnamon Twig, 10 parts by weight of Chinese Knotweed and 15 parts by weight of Commelina Communis.

The raw materials were mixed and pulverized into decoction pieces. The decoction pieces were dissolved in 60% ethanol, and the volume of the ethanol was 10 folds of the mass of the decoction pieces. The decoction pieces were decocted at 70°C for 2 hours, filtered to give a filter residue for later use. A first filtrate was collected. The filter residue was decocted 3 times with 60% ethanol at 70°C, the volume of which was 10 folds of the mass of the filter residue, 2 hours for each time, and a second filtrate was obtained. The first filtrate and the second filtrate were mixed and concentrated at 60°C under reduced pressure to give the pharmaceutical cream with a relative density of 1.10-1.40.

Preparation of gel dosage form: A mixture of polyhexamethylene biguanide and carbomer was soaked in purified water for 4 hours, thereby forming a gel matrix for later use. To the gel matrix, the pharmaceutical cream, glycerin and azone were added, and then purified water was added. Triethanolamine was added dropwise to the gel matrix to adjust pH = 7, and stirred to give a gel dosage form.

The pharmaceutical cream is not limited to the gel dosage form. A suppository or other dosage forms can be prepared by mixing glycerin and an excipient to a suppository mold.

### Example 2

Example 2 is basically the same as Example 1 except that the traditional Chinese medicine composition comprises following raw materials: 30 parts by weight of smilax glabra root, 15 parts by weight of Cinnamon Twig, 10 parts by weight of Chinese Knotweed and 15 parts by weight of Commelina Communis.

The raw materials were mixed and pulverized into decoction pieces. The decoction pieces were dissolved in 70% ethanol, and the volume of the ethanol was 10 folds of the mass of the decoction pieces. The decoction pieces were decocted at 75°C for 1.5 hours, filtered to give a filter residue for later use. A first filtrate was collected. The filter residue was decocted 4 times with 75% ethanol at 70°C, the volume of which was 10 folds of the mass of the filter residue, 1.5 hours for each time, and a second filtrate was obtained. The first filtrate and the second filtrate were mixed and concentrated at 60°C under reduced pressure to give the pharmaceutical cream with a relative density of 1.35.

An excipient was added to the pharmaceutical cream to prepare a suppository. Specifically, to 10 g of the pharmaceutical cream, the excipient containing 60 g of glycerin and 55 g of polyethylene glycol was added. The mixture was melted, poured into a suppository mold, cooled, removed from the mold and packed individually to obtain a suppository.

### Example 3

A traditional Chinese medicine composition comprises following raw materials: 55 parts by weight of smilax glabra root, 13 parts by weight of Cinnamon Twig, 15 parts by weight of Chinese Knotweed and 18 parts by weight of Commelina Communis.

The raw materials were mixed and pulverized into decoction pieces. The decoction pieces were dissolved in 60% ethanol, and the volume of the ethanol was 8 folds of the mass of the decoction pieces. The decoction pieces were decocted at 55°C for 4 hours, filtered to give a filter residue for later use. A first filtrate was collected. The filter residue was decocted 5 times with 60% ethanol at 55°C, the volume of which was 8 folds of the mass of the filter residue, 3 hours for each time, and a second filtrate was obtained. The first filtrate and the second filtrate were mixed and concentrated at 60°C under reduced pressure to give the pharmaceutical cream with a relative density of 1.3.

To 100 g of the pharmaceutical cream, 1 L of water, 30 g of polysorbate-80, and 5 g of sodium benzoate were added and mixed. The mixture was packed individually to obtain a lotion.

### Example 4

A traditional Chinese medicine composition comprises following raw materials: 50 parts by weight of smilax glabra root, 13 parts by weight of Cinnamon Twig, 18 parts by weight of Chinese Knotweed and 12 parts by weight of Commelina Communis.

The raw materials were mixed and pulverized into decoction pieces. The decoction pieces were dissolved in 60% ethanol, and the volume of the ethanol was 8 folds of the mass of the decoction pieces. The decoction pieces were decocted at 55°C for 4 hours, filtered to give a filter residue for later use. A first filtrate was collected. The filter residue was decocted 5 times with 60% ethanol at 55°C, the volume of which was 8 folds of the mass of the filter residue, 3 hours for each time, and a second filtrate was obtained. The first filtrate and the second filtrate were mixed and concentrated at 60°C under reduced pressure to give the pharmaceutical cream with a relative density of 1.2.

To the gel matrix, a mixed solution containing 150 g of the pharmaceutical cream and 50 g of glycerol, 20 g of azone was added. Thereafter, purified water was added, and triethanolamine was added dropwise to the gel matrix to adjust pH = 7. The mixture was stirred and packed individually to give a gel dosage form.

### Example 5

A traditional Chinese medicine composition for treatment of HPV infection comprises following raw materials: 30 parts by weight of smilax glabra root, 15 parts by weight of Cinnamon Twig, 15 parts by weight of Chinese Knotweed and 15 parts by weight of Commelina Communis.

The raw materials were mixed, pulverized into decoction pieces, and dissolved in 60% ethanol, and the volume of the ethanol was 10 folds of the mass of the decoction pieces. The decoction pieces were filtered to give a filter residue for later use. A first filtrate was collected. The residue was dissolved in 60% ethanol, the volume of which was 10 folds of the mass of the filter residue, filtered to yield a second filtrate. The filtrates were obtained 3 times through reflux extraction, 2 hours for each time. The first filtrate and the second filtrate were mixed and concentrated at 60°C under reduced pressure to give the pharmaceutical cream with a relative density of 1.2.

1 L of water, 50g of polysorbate-80, and 5 g of sodium benzoate were added to 100 g of the pharmaceutical cream and mixed. The mixture was packed individually to obtain a lotion.

### Example 6

A traditional Chinese medicine composition for treatment of HPV infection comprises following raw materials: 30 parts by weight of smilax glabra root, 10 parts by weight of Cinnamon Twig, 10 parts by weight of Chinese Knotweed and 10 parts by weight of Commelina Communis.

The raw materials were mixed and pulverized into decoction pieces. The decoction pieces were dissolved in water, the volume of which was 12 folds of the mass of the decoction pieces, filtered to give a filter residue for later use. A first filtrate was collected. The filter residue was extracted 2 times through reflux extraction, 2 hours for each time, to yield a second filtrate. The first filtrate and the second filtrate were mixed, absorbed using an AB-8 macroporous absorbent resin, and eluted consecutively with water and 75% ethanol. The eluent comprising 75% ethanol was collected and concentrated at 60°C under reduced pressure to give the pharmaceutical cream with a relative density of 1.2.

5 g of dried pharmaceutical cream at 60°C was mixed with an excipient containing 70 g of glycerin and 50 g of polyethylene glycol. The mixture was melted, poured into a suppository mold, cooled, removed from the mold and packed individually to obtain a suppository.

### Example 7

A traditional Chinese medicine composition for treatment of HPV infection comprises following raw materials: 45 parts by weight of smilax glabra root, 10 parts by weight of Cinnamon Twig, 15 parts by weight of Chinese Knotweed and 10 parts by weight of Commelina Communis.

The raw materials were mixed and pulverized into decoction pieces. The decoction pieces were dissolved in 60% ethanol, the volume of which was 10 folds of the mass of the decoction pieces, and filtered to give the filtrate. The filtrates were obtained 2 times through reflux extraction, 2 hours for each time. The filtrates were mixed, absorbed using the AB-8 macroporous absorbent resin, and eluted consecutively with water and 75% ethanol. The eluent comprising 75% ethanol was collected and concentrated at 60°C under reduced pressure to give the pharmaceutical cream with a relative density of 1.3.

Preparation of gel dosage form: 500 mL of purified water was added to a mixture of 15 g of polyhexamethylene biguanide and 3 g of carbomer. The mixture was soaked in water for 4 hours, thereby forming a gel matrix for later use. To the gel matrix, a mixed solution containing 150 g of the pharmaceutical cream, 50 g of glycerin, and 10 g of azone was added, followed by addition of 1000 mL of purified water. Triethanolamine was added dropwise to the gel matrix to adjust pH = 7, and stirred to give a gel dosage form.

### Example 8

### The traditional Chinese medicine composition inhibits the proliferation of Hela cells

The percentage inhibition on HeLa cell proliferation for each compound prepared in Examples 1-7 was determined by MTT assay (MTT Cell Proliferation and Cytotoxicity Assay Kit). HeLa cells was cultured in a 96-well plate for 6.0×10³ cells per well, and allowed to adhere to the surface of the 96-well plate. Serum-free medium was added to each well of control group. Cidofovir was diluted in purified water such that the final concentration of cidofovir in the assay was 10 µmol/mL, and the resulting solution was added to each well of positive control group. Each compound of Examples 1-7 was diluted in water to a crude drug concentration of 25 g/L, and the resulting solution was added to the 96-well plate for a final volume of 200 µL per well. 200 µL of complete medium was only added to each well of blank control group. Each experimental group was done in 6 well replicates. After incubation of 24, 48, and 72 hours, 20 µL of 5 g/L MTT was added to each well and the plates were further incubated at 37°C for 4 hours. Following discarding the supernatant, 200 µL of dimethyl sulfoxide (DMSO) was added to each well and the plates were shaken sufficiently as so to dissolve the crystals. Absorbance (A) of the wells at 490 nm was measured. These measurements then are used to calculate the percentage inhibition of cell growth: percentage inhibition = 1 - (T_{A} - C_{A}) × 100%, where T_{A} refers to the average absorbance of treatment group, and C_{A} refers to the average absorbance of control group. Each experiment was performed in triplicate, and the average percentage inhibition in each group are shown in Table 1.

**Table 1 Comparison of inhibition percentage of pharmaceutical preparations on HeLa cell proliferation**

| Group | 24 hours | 48 hours | 72 hours |
|---|---|---|---|
| Blank group | 1.56±0.44 | 3.20±1.01 | 4.05±0.52 |
| Cidofovir | 32.24±2.08 | 48.58±3.50 | 58.09±3.21 |
| Example 1 | 42.74±1.48 | 55.83±2.47 | 74.89±2.63 |
| Example 2 | 41.45±1.94 | 54.73±2.91 | 74.75±2.62 |
| Example 3 | 43.82±2.78 | 57.46±2.18 | 77.35±3.11 |
| Example 4 | 41.32±2.03 | 57.74±2.95 | 76.48±2.27 |
| Example 5 | 39.55±2.17 | 56.08±3.24 | 75.84±2.58 |
| Example 6 | 42.17±2.66 | 58.24±1.97 | 78.01±3.06 |
| Example 7 | 43.58±3.04 | 60.62±2.65 | 80.33±3.14 |

The traditional Chinese medicine composition of the disclosure for use in treatment of HPV infection has the ability to inhibit cell proliferation in the HeLa cells. Percentage inhibition increases with the increase of culture time, which indicates an effect that is statistically significant compared to the corresponding control group.

### Example 9

### Evaluation of the efficacy of the compounds in treatment of HPV infection

Compounds prepared in Examples 5-7 was tested and evaluated for efficacy. The volunteers infected with high-risk HPV were randomly divided into equal groups, and each group contained 50 people. A suppository comprising a blank matrix was delivered to the cervix of each volunteer in blank control group. A suppository comprising an interferon was delivered to the cervix of each volunteer in positive control group. The lotion prepared in Example 5 was used to clean the area surrounding the labia and inside the vagina. The suppository prepared in Example 6 and the gel dosage form prepared in Example 7 were delivered to the cervix, respectively. After 12 weeks of drug treatment, each volunteer got a physical examination involving recording the symptoms that the volunteers feel subjectively, gynecological examination, and microscopic examination of vaginal discharge, so as to analysis and evaluate the efficacy of the compounds in treatment of patient with high-risk HPV. A negative HPV test means that the volunteer is cured. A reduction in positive viral load greater than (or equal to 50%) is considered to be effective (Digene HC2 HPV DNA Test), otherwise, the drug is invalid against HPV. The results are shown in Table 2 below.

**Table 2 Comparison of clinical efficacy of pharmaceutical preparations on HPV**

| Group | Cases | Cured | effective | invalid | Total effective rate/% |
|---|---|---|---|---|---|
| Blank control group | 50 | 0 | 0 | 50 | 0 |
| Positive control group | 50 | 6 | 30 | 14 | 72 |
| Example 5 | 50 | 34 | 9 | 7 | 86 |
| Example 6 | 50 | 39 | 6 | 5 | 90 |
| Example 7 | 50 | 38 | 8 | 4 | 92 |

Result of Table 2 show that the total effective rate of the traditional Chinese medicine composition in treatment of high-risk HPV infection is high than 86%, in which the cure rate is higher than 68%.

During the 6-month experiment period, by building an international standardized comparison model and performing a series of experiments for pharmacological and pathological tests, the traditional Chinese medicine composition of the disclosure provides superior capability for identification and inactivation of HPV in the cells, and has ability to kill the host HeLa cell of cervical cancer caused by HPV, especially the high-risk mucosal HPV types.

### Example 10

### Clinical case I

A 32-year-old female was diagnosed with HPV33 in May 2016 and failed to respond to treatment with interferon. Patient still tested positive for HPV33 in October 2017 and was coinfected with HPV18, 52, and 56. Colposcopy and directed biopsies (CDB) was performed and showed CIN1. From December 2017 to March 2018, the lotion prepared in Example 5 was used for 5 consecutive days followed by 2 consecutive days of rest, for nine seven-day cycles. Note no treatment during menstrual periods. Then HPV tested negative.

### Example 11

### Clinical case II

A 62-year-old female was diagnosed with HPV58 in September 2011 and the infection had persisted for more than 6 years. Patient still tested positive for HPV58 in September 2017 and was coinfected with HPV81. From December 2017 to March 2018, the suppository prepared in Example 6 was used for 5 consecutive days followed by 2 consecutive days of rest, for nine seven-day cycles. Note no treatment during menstrual periods. The patient was tested negative for HPV43, 51, and 83 in September 2018.

### Example 12

### Clinical case III

A59-year-old female was diagnosed with HPV for half a year after 10 years after hysterectomy. Patient failed to respond to treatment with a gel dosage form obtained from a TCM company. From June 2018 to August 2018, the gel dosage form prepared in Example 7 of the disclosure was used for 5 consecutive days followed by 2 consecutive days of rest, for nine seven-day cycles. Note no treatment during menstrual periods. HPV43, 51, and 83 tested negative in November 2018.

In conclusion, the traditional Chinese medicine composition of the disclosure has the ability to kill HPV and human cervical cancer HeLa cells, and to treat cervicitis, which is suitable for treatment of HPV infection and the associated diseases, such as vaginitis, cervicitis, and cervical ectropion. The TCM composition has a mild smell safe for human body and acts with mil side effects, which can directly act on the vagina without irritation. The one-time bolus injection of gel dosage form, suppository and lotion dosage form are easy for patients to use and improve patient compliance. The drugs in above dosage form can remain in the vagina for a long time and penetrate into the mucosal folds, which helps the drug to contact the lesion and pathogenic factors, and facilitates the drug absorption.

## Claims

1. A pharmaceutical composition, comprising an extract of:
30-60 parts by weight of Smilax Glabra Root;
10-20 parts by weight of Cinnamon Twig;
10-20 parts by weight of Chinese Knotweed; and
10-20 parts by weight of Commelina Communis.

2. The pharmaceutical composition of claim 1, comprising an extract of:
30-55 parts by weight of Smilax Glabra Root;
12-18 parts by weight of Cinnamon Twig;
10-18 parts by weight of Chinese Knotweed; and
12-18 parts by weight of Commelina Communis.

3. A pharmaceutical preparation, comprising the pharmaceutical composition of claim 1 or 2.

4. The pharmaceutical preparation of claim 3, having a dosage form of creams, gels, or suppositories.

5. The pharmaceutical preparation of claim 3, having a dosage form of lotions.

6. A method of preparing a pharmaceutical preparation, the method comprising: evenly mixing raw materials of the pharmaceutical composition of claim 1 or 2, extracting, and concentrating an extract to yield a cream.

7. The method of claim 6, comprising:
1) mixing the raw materials to yield a mixture;
2) dissolving the mixture in an ethanol solution, decocting the mixture dissolved in the ethanol solution, thus yielding a first filtrate and a first residue;
3) collecting the first filtrate, dissolving the first residue in the ethanol solution, and decocting the first residue dissolved in the ethanol solution, thus yielding a second filtrate and a second residue; and
4) repeating 3), mixing collected filtrates, and condensing the filtrates to yield the cream having a relative density of 1.10-1.40 at 60°C.

8. The method of claim 7, wherein the ethanol solution has a concentration of 50-80% (v/v), and a volume weight ratio of the ethanol solution to the mixture is 5-15.

9. The method of claim 6, comprising:
a) mixing the raw materials to yield a mixture;
b) dissolving the mixture in water, decocting the mixture dissolved in the water, thus yielding a first filtrate and a first residue;
c) collecting the first filtrate, dissolving the first residue in the water, and decocting the first residue dissolved in the water, thus yielding a second filtrate and a second residue;
d) repeating c), mixing collected filtrates, and condensing the filtrates to yield the cream having a relative density of 1.10-1.40 at 60°C.

10. The method of claim 7, wherein the mixture and the filtrates are decocted in the ethanol solution at 50-75°C for 2-5 hours.

11. The method of claim 7, wherein repeating 3) for 2-5 times, and the filtrates are condensed at 50-80°C.

12. The method of any one of claims 7-11, further comprising adding an excipient to the cream to yield a gel or suppository.

13. The method of any one of claims 7-11, further comprising adding an excipient to the cream to yield a lotion.

14. A pharmaceutical preparation of any one of claims 3-5 for prevention and/or treatment of human papillomavirus (HPV) infection.

15. A pharmaceutical preparation of any one of claims 3-5 for prevention and/or treatment of vaginitis, cervicitis, or cervical ectropion.

## Patentansprüche

1. Pharmazeutische Zusammensetzung, umfassend einen Extrakt aus:
30-60 Gewichtsteilen Smilax-Glabra-Wurzel;
10-20 Gewichtsteilen Zimtzweig;
10-20 Gewichtsteilen Chinesischer Knöterich; und
10-20 Gewichtsteilen Commelina Communis.

2. Pharmazeutische Zusammensetzung nach Anspruch 1, umfassend einen Extrakt aus:
30-55 Gewichtsteilen Smilax-Glabra-Wurzel;
12-18 Gewichtsteilen Zimtzweig;
10-18 Gewichtsteilen Chinesischer Knöterich; und
12-18 Gewichtsteilen Commelina Communis.

3. Pharmazeutische Zubereitung, umfassend die pharmazeutische Zusammensetzung nach Anspruch 1 oder 2.

4. Pharmazeutische Zubereitung nach Anspruch 3 mit einer Dosierungsform von Cremes, Gelen oder Zäpfchen.

5. Pharmazeutische Zubereitung nach Anspruch 3 mit einer Dosierungsform von Lotionen.

6. Verfahren zum Zubereiten einer pharmazeutischen Zubereitung, wobei das Verfahren Folgendes umfasst: gleichmäßiges Mischen von Rohmaterialien der pharmazeutischen Zusammensetzung nach Anspruch 1 oder 2, Extrahieren und Konzentrieren eines Extrakts, um eine Creme zu erhalten.

7. Verfahren nach Anspruch 6, umfassend:
1) Mischen der Rohmaterialien, um eine Mischung zu erhalten;
2) Auflösen der Mischung in einer Ethanollösung, Auskochen der in der Ethanollösung aufgelösten Mischung, wodurch ein erstes Filtrat und ein erster Rückstand erhalten werden;
3) Sammeln des ersten Filtrats, Auflösen des ersten Rückstands in der Ethanollösung und Auskochen des in der Ethanollösung aufgelösten ersten Rückstands, wodurch ein zweites Filtrat und ein zweiter Rückstand erhalten werden; und
4) Wiederholen von 3), Mischen von gesammelten Filtraten und Kondensieren der Filtrate, um die Creme mit einer relativen Dichte von 1,10-1,40 bei 60 °C zu erhalten.

8. Verfahren nach Anspruch 7, wobei die Ethanollösung eine Konzentration von 50-80 % (v/v) aufweist und ein Volumengewichtsverhältnis der Ethanollösung zu der Mischung 5-15 ist.

9. Verfahren nach Anspruch 6, umfassend:
a) Mischen der Rohmaterialien, um eine Mischung zu erhalten;
b) Auflösen der Mischung in Wasser, Auskochen der in dem Wasser aufgelösten Mischung, wodurch ein erstes Filtrat und ein erster Rückstand erhalten werden;
c) Sammeln des ersten Filtrats, Auflösen des ersten Rückstands in dem Wasser und Auskochen des in dem Wasser aufgelösten ersten Rückstands, wodurch ein zweites Filtrat und ein zweiter Rückstand erhalten werden;
d) Wiederholen von c), Mischen von gesammelten Filtraten und Kondensieren der Filtrate, um die Creme mit einer relativen Dichte von 1,10-1,40 bei 60 °C zu erhalten.

10. Verfahren nach Anspruch 7, wobei die Mischung und die Filtrate in der Ethanollösung bei 50-75 °C für 2-5 Stunden ausgekocht werden.

11. Verfahren nach Anspruch 7, wobei 3) 2-5-mal wiederholt wird und die Filtrate bei 50-80 °C kondensiert werden.

12. Verfahren nach einem der Ansprüche 7-11, ferner umfassend das Hinzufügen eines Hilfsstoffes zu der Creme, um ein Gel oder Zäpfchen zu erhalten.

13. Verfahren nach einem der Ansprüche 7-11, ferner umfassend das Hinzufügen eines Hilfsstoffes zu der Creme, um eine Lotion zu erhalten.

14. Pharmazeutische Zubereitung nach einem der Ansprüche 3-5 zur Vorbeugung und/oder Behandlung einer Infektion mit humanem Papillomvirus (HPV).

15. Pharmazeutische Zubereitung nach einem der Ansprüche 3-5 zur Vorbeugung und/oder Behandlung von Vaginitis, Zervizitis oder Zervixerosion.

## Revendications

1. Composition pharmaceutique, comprenant un extrait de :
30 à 60 parties en poids de racine de Smilax glabra ;
10 à 20 parties en poids de brindilles de cannelle ;
10 à 20 parties en poids de renouée de Chine ; et
10 à 20 parties en poids de comméline commune.

2. Composition pharmaceutique selon la revendication 1, comprenant un extrait de :
30 à 55 parties en poids de racine de Smilax glabra ;
12 à 18 parties en poids de brindilles de cannelle ;
10 à 18 parties en poids de renouée de Chine ; et
12 à 18 parties en poids de comméline commune.

3. Préparation pharmaceutique, comprenant la composition pharmaceutique selon la revendication 1 ou 2.

4. Préparation pharmaceutique selon la revendication 3, présentant une forme posologique de crèmes, de gels ou de suppositoires.

5. Préparation pharmaceutique selon la revendication 3, présentant une forme posologique de lotions.

6. Procédé de préparation d'une préparation pharmaceutique, le procédé comprenant : le mélange homogène des matières premières de la composition pharmaceutique selon la revendication 1 ou 2, l'extraction et la concentration d'un extrait pour produire une crème.

7. Procédé selon la revendication 6, comprenant :
1) le mélange des matières premières pour produire un mélange ;
2) la dissolution du mélange dans une solution d'éthanol, la décoction du mélange dissous dans la solution d'éthanol, produisant ainsi un premier filtrat et un premier résidu ;
3) la collecte du premier filtrat, la dissolution du premier résidu dans la solution d'éthanol, et la décoction du premier résidu dissous dans la solution d'éthanol, produisant ainsi un second filtrat et un second résidu ; et
4) la répétition de 3), le mélange des filtrats collectés et la condensation des filtrats pour produire la crème présentant une densité relative de 1,10 à 1,40 à 60°C.

8. Procédé selon la revendication 7, ladite solution d'éthanol présentant une concentration de 50 à 80 % (v/v), et le rapport volume/poids de la solution d'éthanol au mélange étant de 5 à 15.

9. Procédé selon la revendication 6, comprenant :
a) le mélange des matières premières pour produire un mélange ;
b) la dissolution du mélange dans l'eau, la décoction du mélange dissous dans l'eau, produisant ainsi un premier filtrat et un premier résidu ;
c) la collecte du premier filtrat, la dissolution du premier résidu dans l'eau, et la décoction du premier résidu dissous dans l'eau, produisant ainsi un second filtrat et un second résidu ;
d) la répétition de c), le mélange des filtrats collectés et la condensation des filtrats pour produire la crème présentant une densité relative de 1,10 à 1,40 à 60°C.

10. Procédé selon la revendication 7, ledit mélange et lesdits filtrats étant décoctés dans la solution d'éthanol à une température de 50 à 75°C pendant 2 à 5 heures.

11. Procédé selon la revendication 7, ladite répétition de 3) 2 à 5 fois, et lesdits filtrats étant condensés à une température de 50 à 80°C.

12. Procédé selon l'une quelconque des revendications 7 à 11, comprenant en outre l'ajout d'un excipient à la crème pour produire un gel ou un suppositoire.

13. Procédé selon l'une quelconque des revendications 7 à 11, comprenant en outre l'ajout d'un excipient à la crème pour produire une lotion.

14. Préparation pharmaceutique selon l'une quelconque des revendications 3 à 5 destinée à la prévention et/ou au traitement d'une infection à papillomavirus humain (HPV).

15. Préparation pharmaceutique selon l'une quelconque des revendications 3 à 5 destinée à la prévention et/ou au traitement de la vaginite, de la cervicite ou de l'ectropion cervical.
